# EUROPEAN PATENT SPECIFICATION

(11) **EP 2 510 963 B1**
(45) Date of publication and mention of the grant of the patent: **29.03.2017**
(21) Application number: 11003182.0
(22) Date of filing: 15.04.2011
(51) Int. Cl.: A61M 5/142

(54) **Device for moving a piston inside a cartridge**
Vorrichtung zum Bewegen eines Kolbens in einer Kartusche
Dispositif de déplacement de piston dans une cartouche

(43) Date of publication of application: 17.10.2012
(73) Proprietor: F. Hoffmann-La Roche AG, 4070 Basel (CH); Roche Diabetes Care GmbH, 68305 Mannheim (DE)
(72) Inventor: Moia, Franco, 4402 Frenkendorf (CH); Mueller, Marcel, 9014 St. Gallen (CH); Ducret, Maurice, 3400 Burgdorf (CH)
(74) Representative: Rentsch Partner AG

(56) References cited:
- EP-A1- 0 170 009
- EP-A1- 1 484 071
- EP-A1- 1 779 830
- WO-A2-2005/002652
- FR-A5- 2 091 684
- US-A- 5 336 189

## Description

The present invention relates to a device for moving the piston inside a cartridge, to a system comprising the device as well as to a method using the device according to the preambles of the independent claims.

A typical problem when dispensing or dosing a liquid from a cartridge, e.g. when dosing insulin from an insulin cartridge with an automated insulin pump, is not only to make sure that, for dispensing the liquid, the piston can be pushed forward inside the cartridge body in a controlled manner, but also that the piston cannot be sucked into the cartridge body by a pressure differential between the front and back side of the piston.

Furthermore, when filling the cartridge with a liquid from a liquid reservoir by retracting the piston inside the cartridge body, a further problem is to make sure that, even though a maximum stroke of the piston can be utilized, there is no risk that the piston is drawn out of the cylinder body.

From the prior art, different approaches to solve these problems are known:
For dispensing or dosing liquid from a cartridge or for filling a cartridge with a liquid by means of an automated device it is know to fix the cartridge body inside the device and to connect the piston with a threaded connection to a piston rod of the device by which it can be pushed forward and/or be retracted in a controlled manner inside the cartridge body within the limits of its design stroke.

For manually filling cartridges or syringe assemblies it is furthermore known from the prior art to provide a physical stop for the piston at the end of the cartridge body or of the cylinder of the syringe assembly which prevents the piston from being drawn out of the cartridge body or syringe cylinder.

Furthermore, cartridges or syringe assemblies are known having a piston which provides, at its backside, moveable coupling means which, in a positive manner, engage an especially adapted piston rod once this piston rod is pushed towards the backside of the piston and, once coupled to the piston rod, release the piston rod only in case they are retracted by the piston rod past a defined position within the cartridge body or syringe cylinder.

Furthermore, from EP 0 337 252 A1, a cartridge is know having a piston which provides, at its backside, a threaded pin for coupling it to a piston rod, wherein this threaded pin is formed by an element which is disconnected from the piston once the piston is retracted into a defined position within the cartridge body by a piston rod threaded onto the threaded pin.

The EP 0170009 A1 discloses an angiographic injector for alternative injection out of two syringes. In an embodiment, latch member hook portions are present for releasably coupling to a syringe plunger in such a way that in both directions of the syringe there exists a positive connection, with establishing and releasing the coupling being controlled via an electromagnet.

However, the known solutions using a threaded connection for coupling the piston to a piston rod suffer from the disadvantage that they require a complex relative motion between the piston and the piston rod for establishing and releasing the connection, which is undesirable since it requires certain skills of the user or, in case of an automated device, a complex mechanical arrangement for controlling the motion of the piston rod. Also, there is the disadvantage that the quality of the connection to be established and the possibility to release it later on is influenced by the friction between the piston and the cartridge body or the syringe cylinder, since this friction defines the torque which can be applied to the threaded connection.

The solutions providing a physical stop for the piston at the end of the cartridge body or of the cylinder of the syringe assembly have the disadvantage that in the manufacturing of the cartridge or syringe assembly, additional operating steps are required, which increases the manufacturing costs.

The solutions providing moveable coupling means at the backside of the piston which release the piston rod once the piston is retracted into a defined position or providing an element for connection with the piston rod which element is disconnected from the piston once the piston is retracted into a defined position have the disadvantage that the piston is of complex design and as a result is difficult to manufacture and expensive.

Thus, all of the know solutions suffer from the disadvantage, that they require relatively expensive cartridge or syringe assembly designs that reduce the attractiveness to use them in fields where the cartridge or syringe assembly should be an inexpensive single use article, like e.g. a self filled insulin cartridge for the therapy of diabetes.

Hence, it is the object of the invention to provide technical solutions which at least partially avoid the disadvantages of the prior art.

This object is achieved by the device for moving the piston inside a cartridge, by the system and by the method according to the independent claims.

A first aspect of the invention concerns a device for moving the piston inside a cartridge having a cartridge body and a piston arranged therein that is displaceable along a longitudinal axis of the cartridge body. Such cartridges generally can be described as liquid tight containers comprising a container body with a cylindrical interior space defining a longitudinal axis and with a piston arranged in said interior space, which in a liquid tight manner abuts to the boundary walls of the interior space and is moveable within said interior space along said longitudinal axis in order to increase or reduce the volume of the fluid tight space defined by the boundary walls of the interior space and by the front face of the piston.

The device comprises a first portion and a second portion.

The first portion comprises first coupling means which are adapted for releasably coupling the cartridge body to it in such a manner that in both directions of said longitudinal axis of the cartridge body there exists a positive connection and/or a frictional connection between the cartridge body and the first portion.

The second portion comprises second coupling means for releasably coupling the piston of the cartridge to it in such a manner that in both directions of said longitudinal axis of the cartridge body there exists a positive connection and/or a frictional connection between the piston and the second portion.

The connections which can be established with the first and second coupling means between the first portion and the cartridge body and between the piston and the second portion in each case have to be greater than the frictional forces between the piston and the boundary walls of the cartridge body when moving the piston inside the cartridge, and additionally the forces generated in the filling and/or dispensing operation of the cartridge by pressure differentials established between the front and back side of the piston.

The first portion and the second portion are moveable relative to each other in such a manner that when, in the intended use of the device, e.g. filling of a cartridge with a liquid or dispensing liquid from a cartridge containing a liquid, the cartridge body of a cartridge is coupled via the first coupling means in the above described manner to the first portion and the piston of said cartridge is coupled via the second coupling means in the above described manner to the second portion, a forward and backward displacement of the piston along said longitudinal axis of the cartridge body can be effected by their relative movement.

The second coupling means are designed in such a manner that for establishing and releasing the positive connection and/or frictional connection with the piston, their shape can reversibly be changed.

Said second portion is moveable relative to the first portion forward and backward between a fully retracted position and a fully extended position along a longitudinal axis in order to effect, in the intended use of the device, a forward and backward displacement of the piston inside the cartridge body. The shape of the second coupling means is automatically changed when the second portion is moved into the fully retracted position and/or is moved out of the fully retracted position.

By this design of the device it becomes possible to generate a system according to a second aspect of the invention comprising the device according to the first aspect of the invention and a cartridge especially adapted to be releasably coupled to the first and second coupling means of the device in the described manner, wherein the cartridge can be of a simple, inexpensive design, thus making the system attractive for use in fields where the cartridge should be an inexpensive single use article, like e.g. for use as a system for filling self filled insulin cartridges for the therapy of diabetes or as a system for dosing or dispensing, respectively, insulin from a single use cartridge.

In a preferred embodiment, the device is suitable for use in filling the cartridge with a liquid, preferably with a liquid drug, more preferably with insulin. In this embodiment, the first and second portions of the device are moveable relative to each other in such a manner that, in the intended use of the device, e.g. for filling the cartridge with a liquid, they can effect a forward and a backward displacement of the piston coupled to the second portion along the longitudinal axis of the cartridge body inside the cartridge body coupled to the first portion.

Preferably, the device has drive means for moving the first and second portions relative to each other, like e.g. a battery powered electric motor with a dedicated gear and linkage. Such devices can be automated and thus allow convenient use without specialized skills.

In a further preferred embodiment of the device the second coupling means are designed in such a manner that their change in shape can partially or solely be effected through a contact with the piston and/or with the cartridge body of the cartridge, e.g. through a con-tact force which acts on a linkage mechanism of the second coupling means which, as a result, changes the shape of the second coupling means or e.g. through an electrical contact switch which initiates a change in shape of the second coupling means through an electrically driven mechanism. This coupling concept has the advantage, that the connection between the piston and the second coupling means is established when they are brought into a specific position relative to each other and thus there is no need to separately identify the position of the piston and the second portion for establishing said connection.

In still a further preferred embodiment of the device, the second coupling means are designed in such a manner that their change in shape can partially or solely be effected through control means of the device, like e.g. through an electrically driven linkage mechanism which in dependency of the position of the second portion relative to the first portion changes the shape of the second coupling means. This concept has the advantage, that the connection between the piston and the second coupling means can be established independently of a prior contact between the piston or the cartridge body and the second coupling means and thus without loading the piston or the cartridge body with any forces through the second coupling means before establishing the connection.

Preferably, the device is designed in such a manner that the shape of the second coupling means can or can only be changed when the first and the second portions assume a specific position relative to each other. This functionality makes sure that the connection between the second portion of the device and the piston of the cartridge can or can only be established and be released in specific relative positions of the first and second portions relative to each other, e.g. can or can only be established and released in relative positions which correspond to a fully retracted position of the piston of the cartridge received in the device for filling, which would be useful in devices which are intended for filling cartridges, or e.g. can or can only be established in a relative position which corresponds to a fully retracted position of the piston of the cartridge received in the device for dispensing liquid from it and can or can only be released in a relative position which corresponds to a fully forwards pushed position of the piston of said cartridge, which would be useful in devices for dispensing a liquid from a cartridge.

If the device is designed in such a manner that the shape of the second coupling means is automatically changed when the first and the second portions are moved into a specific position relative to each other and/or are moved out of a specific position relative to each other, which is preferred, the advantage is arrived at that the device has a uncomplicated control concept and is easy to understand and operate even for unskilled person.

In order to make sure that a release of the first coupling means and a subsequent removal of the cartridge body from the first portion cannot result in the piston being pulled out of the cartridge body, it is furthermore preferred that the device is designed in such a manner that the shape of the second coupling means can or can only be changed into a coupled or engaging status when the cartridge body of a cartridge has been coupled to the first portion by means of the first coupling means and/or that the shape of the second coupling means is automatically changed into a released status when, after the cartridge body of a cartridge has been coupled to the first portion by means of the first coupling means, said first coupling means are released.

In still a further preferred embodiment of the device, the device can be designed in such a manner that the shape of the second coupling means automatically changes from a releasing status into a coupling or engaging status when, starting from the fully retracted position, a forward movement of the second portion is initiated, and is changed back to the releasing status when the second portion, after the forward movement, again reaches the fully retracted position. Such devices are especially suitable as filling devices for self filled cartridges.

In still a further preferred embodiment of the device, the second coupling means comprise a pincer mechanism having at least two pincer arms, the shape of which can be changed through a closing and opening movement of the pincer arms. The pincer arms provide protrusions extending away from them for engagement into radial undercuts formed in a piston of a cartridge to be filled. By means of such a pincer mechanism, second coupling means that can change their shape in order to establish and release a connection with a piston of a cartridge can in a reliable and cost effective manner be made available.

Preferably, said pincer mechanism can be opened and closed by control means of the device which, for doing so, are brought into contact with contact surfaces of the pincer arms and are moved relative to these contact surfaces in direction of the longitudinal axis of the cartridge body to open or to close the pincer mechanism. In this manner, the pincer mechanism can be actuated in a simple way and with a minimum of additional moveable parts.

In the before described embodiment it is furthermore prefer-red that device is designed in such a manner that the pincer mechanism is guided in guides for linear movement and can be moved forwards and backwards by means of a cam wheel which can be rotated by drive means.

The linkage between the cam wheel and the pincer mechanism preferably is realized in that the pincer mechanism comprises a pin or key which travels in a groove formed in the cam wheel. Such a linkage for generating a movement of the second portion relative to the first portion is simple in design, inexpensive in manufacturing and reliable in operation.

If furthermore the pincer mechanism can be opened and closed by control means of the device which are as well operated by the cam wheel, which is preferred, a forced mechanical coupling between the movement of the first and second portions relative to each other and the change in shape of the second coupling means results.

Preferably, these control means for opening and closing the pincer mechanism comprise a pusher which can be pushed up by a notch formed at the cam wheel, thereby pushing together the arms of the pincer mechanism. By this design, a simple and reliable mechanism for controlling the change in shape of the second coupling means and the relative movement between the first and second portions of the device results.

Furthermore, in the before mentioned embodiments in which both, the relative movement between the first and second portions as well as the opening and closing of the pincer mechanism is controlled by a common cam wheel, it is preferred that the cam wheel is designed in such a manner that, while the pincer mechanism is opened and closed by control means operated by the cam wheel, the pincer mechanism which is part of the second portion of the device is held by the cam wheel in a fully retracted position. By means of this control concept it is possible to ensure that the pincer mechanism is coupled to the piston of the cartridge in a defined position.

By advantage, the cam wheel is coupled to a drive unit, e.g. an electrical motor with associated gear and control, via a rotationally flexible coupling. By means of this, if the cam wheel is rotated forwards and backwards between two physical stops, control of the drive unit can be realized without the risk of overloading components due to recent torque peaks by monitoring the energy consumption of the drive unit and by stopping the drive unit or reverse its rotation direction once a specific energy consumption, e.g. in the case of an electric motor a certain electric current, is detected. However, also other control concepts are envisaged, e.g. comprising sensors which are detecting certain relative positions of the first and second portions or certain angles of rotation of the cam wheel.

In a further preferred embodiment, the device according to the first aspect of the invention is a device for filling a cartridge with a liquid from a liquid reservoir or for dispensing or dosing a liquid from a cartridge containing a liquid, preferably a liquid drug, more preferably insulin. In these applications, the advantages of the invention become especially clearly apparent.

A second aspect of the invention concerns a system comprising a device according to the first aspect of the invention and a cartridge having a cartridge body and a piston arranged therein displaceable along a longitudinal axis of the cartridge body.

The cartridge body of the cartridge is especially adapted to be releasably coupled to the first coupling means of the first portion of the device in such a manner that in both directions of said longitudinal axis of the cartridge body there exists a positive connection and/or a frictional connection between the cartridge body and the first portion.

The piston of the cartridge is especially adapted to be releasably coupled to the second coupling means of the second portion in such a manner that in both directions of said longitudinal axis of the cartridge body there exists a positive connection and/or a frictional connection between the piston and the second portion.

The connections which can be established between the first portion and the cartridge body and between the piston and the piston rod in each case are greater than the frictional forces between the piston and the boundary walls of the cartridge body when moving the piston inside the cartridge and the forces generated in the intended use of the system, e.g. filling of the cartridge with a liquid and/or dispensing liquid from the cartridge, by pressure differentials between the front and back side of the piston.

Since in the system according to the second aspect of the invention the cartridge can be of a simple, inexpensive design, the system is attractive for use in fields where the cartridge should be an inexpensive single use article, like e.g. for use as a system for filling self filled insulin cartridges for the therapy of diabetes or as a system for dosing or dispensing, respectively, insulin from a single use cartridge.

In a preferred embodiment of the system, the piston of the cartridge at its back side comprises at least one radial undercut for engagement of the second coupling means of the second portion of the device.

The second coupling means of the device comprise moveable parts and are designed in such a manner that, when the cartridge body is coupled by the first coupling means to the first portion of the device and the second portion is moved towards the piston and starts to displace the piston forwards inside the cartridge body, the moveable parts of the second coupling means are moved, preferably against spring forces, partially or solely through a contact with the piston and/or with the cartridge body, from a position, in which there is no engagement with the at least one radial undercut in the piston into a position in which they engage said at least one radial undercut in such a manner that in both directions of said longitudinal axis of the cartridge body there exists a positive connection between the piston and the second portion enabling a forward and backward displacement of the piston through a movement of the second portion.

Furthermore the second coupling means of the device are designed in such a manner that when, during a subsequent backward displacement of the piston inside the cartridge body effected by the second portion the piston reaches a specific retracted position within the cartridge body, the moveable parts are moved, preferably by spring forces, from the position, in which they engage said at least one radial undercut back into the position in which there is no engagement with the at least one radial undercut.

As mentioned earlier, this coupling concept has the advantage that the connection between the piston and the second coupling means can be established when the piston and the second coupling means are brought into a specific position relative to each other and thus there is no need to separately identify the exact positions of the piston and the second portion for establishing said connection.

In still a further preferred embodiment of the system, again the piston of the cartridge at its back side comprises at least one radial undercut, for engagement of the second coupling means of the second portion of the device.

The second coupling means of the device comprise moveable parts and are designed in such a manner that, when the cartridge body is coupled by the first coupling means to the first portion of the device and when the second portion of the device is arranged in the fully retracted position or in a specific almost fully retracted position and when a forward movement of the second portion is initiated, the moveable parts of the second coupling means, preferably by spring forces, partially or solely controlled by control means of the device, are moved from a position, in which there is no engagement with the at least one radial undercut in the piston, into a position in which they engage said at least one radial undercut in such a manner that in both directions of said longitudinal axis of the cartridge body there exists a positive connection between the piston and the second portion enabling a forward and backward displacement of the piston through a movement of the second portion.

As mentioned earlier, this coupling concept has the advantage that the connection between the piston and the second coupling means can be established independently of a prior contact between the piston and the second coupling means and thus without any forces acting from the second portion onto the piston or the cartridge body before establishing the connection.

Preferably, in this embodiment the second coupling means are designed in such a manner that, when, after the forward movement of the second portion has been initiated, during a subsequent backward displacement of the piston inside the cartridge body effected through a movement of the second portion relative to the first portion, the second portion again reaches the fully retracted position or the specific almost fully retracted position, the moveable parts of the second coupling means are moved, by advantage against spring forces, partially or solely controlled by control means of the device, from the position, in which they engage said at least one radial undercut in the piston back into the position in which there is no engagement with the at least one radial undercut. Such systems are especially suitable as automated filling systems for self filled cartridges.

By advantage, the moveable parts of the second coupling means of the device of the system comprise or are formed by the resilient arms of a pincer mechanism. Such a pincer mechanism are reliable and inexpensive.

Using a device according to the first aspect of the invention, a method of moving the piston in a cartridge having a cartridge body and a piston arranged therein displaceable along a longitudinal axis of the cartridge body can be carried out.

In a first step of the method, a system according to the second aspect of the invention is provided, comprising a device according to the first aspect of the invention and a dedicated cartridge.

Thereafter, the cartridge body of the cartridge and the piston of the cartridge are releasably coupled by the first and the second coupling means of the device to the first and second portions of the device in each case in such a manner that in both directions of the longitudinal axis of the cartridge body there exists a positive connection and/or a frictional connection between the cartridge body and the first portion and between the piston and the second portion. For establishing the connection between the piston and the second portion, the shape of the second coupling means is changed.

After the cartridge body and the piston of the cartridge have been coupled to the first and second portions of the device by the first and second coupling means, the piston is moved inside the cartridge body along the longitudinal axis of same through a relative movement between the first and second portions of the device.

Since in the method the cartridge can be of a simple, inexpensive design, the method is attractive for use in fields where the cartridge should be an inexpensive single use article, like e.g. as method of filling self filled insulin cartridges for the therapy of diabetes or as method of dosing or dispensing, respectively, insulin from a single use cartridge.

In a preferred embodiment of the method, the method is a method of filling a cartridge with a liquid, preferably with a liquid drug, more preferably with insulin, from a liquid reservoir.

In this preferred embodiment, the cartridge, which is coupled with its cartridge body and its piston to the first and second portions of the device by the first and second coupling means as described before, is connected with a liquid reservoir containing a liquid in such a manner that the inside of the liquid reservoir and the inside of the cartridge communicate with each other while they are hermetically sealed against the outside.

After the cartridge has been connected to the liquid reservoir, the piston is moved inside the cartridge body along the longitudinal axis of same through a relative movement between the first and second portions of the device in such a manner that the volume of the inside of the cartridge is increased, thereby displacing liquid from the inside of the liquid reservoir into the inside of the cartridge.

Preferably, before increasing the volume of the inside of the cartridge as described above for displacing liquid from the inside of the liquid reservoir into the inside of the cartridge, the piston is moved inside the cartridge body along the longitudinal axis of same through a relative movement between the first and second portions of the device in such a manner that the volume of the inside of the cartridge is reduced. This can take place according to alternative embodiments of the method before or after the cartridge has been connected to the liquid reservoir.

In another preferred embodiment of the method , the method is a method of dispensing or dosing a liquid, preferably a liquid drug, more preferably insulin, from a cartridge containing the liquid.

In this preferred embodiment, the cartridge of the system according to the second aspect of the invention which is provided contains the liquid to be dosed or dispensed.

After the cartridge body and the piston of the cartridge have been coupled to the first and second portions of the device by the first and second coupling means in the manner described earlier, the piston is moved inside the cartridge body along the longitudinal axis of same through a relative movement between the first and second portions of the device in such a manner that the volume of the inside of the cartridge is reduced, thereby dispensing liquid from the cartridge.

Further preferred embodiments of the invention become apparent from the dependent claims and from the following description by way of the drawings. Therein show:
Fig. 1 a side view of a device according to the invention;
Fig. 2 a sectional view of the device of Fig. 1 along line B-B in Fig. 3; Fig. 3 a sectional view of the device of Fig. 1 along line A-A in Fig. 1;
Fig. 4 the detail X of Fig. 3;
Fig. 5 the detail Y of Fig. 3
Fig. 6 a perspective view from a first direction onto the base plate of the device according to Fig. 1 with the pincer mechanism and the associated drive and control mechanism;
Fig. 7 a perspective view from a second direction onto the pincer mechanism and the associated control mechanism shown in Fig. 6; and
Fig. 8 a) to g) a schematic illustration of an alternative coupling concept according to the invention.

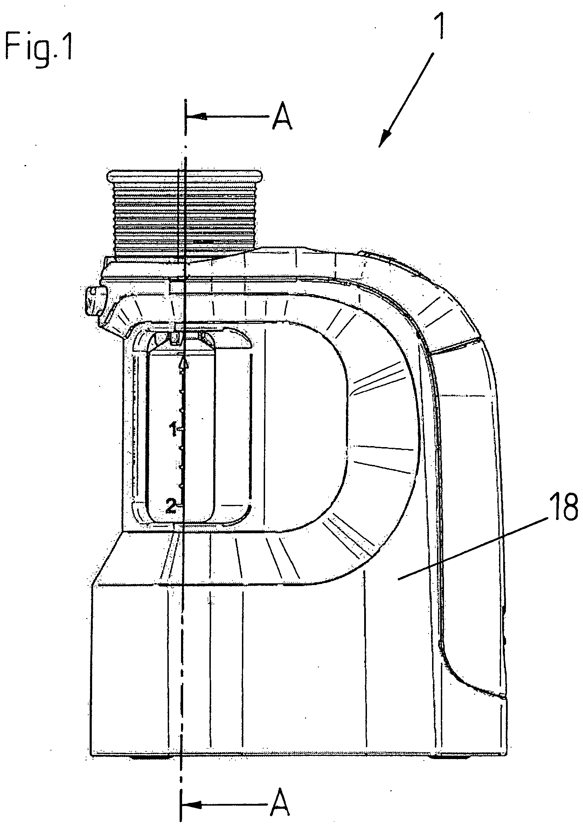

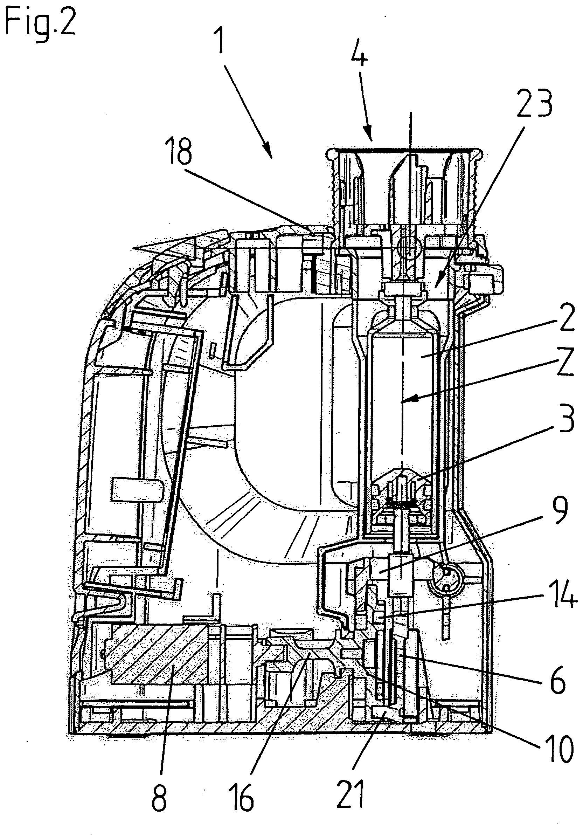

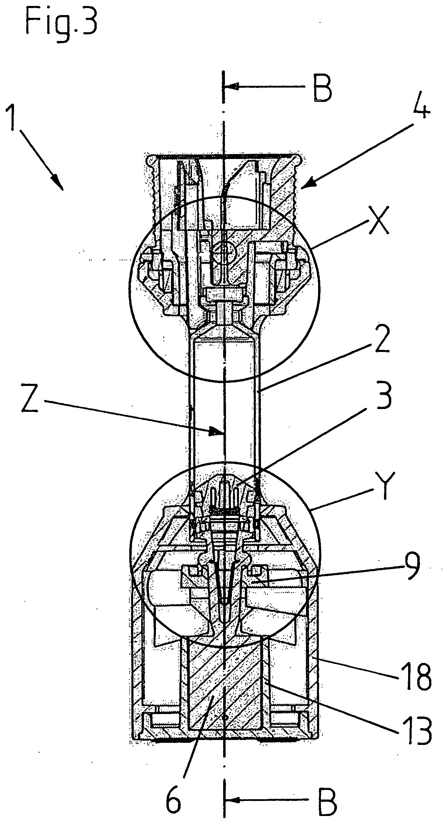

Fig. 1 shows a side view of a device 1 according to the invention for filling self filled insulin cartridges with insulin from an insulin reservoir. The Figures 2 and 3 show sectional views of the device 1, once in section along the line A-A in Fig. 1 (Fig. 3) and once in section along the line B-B in Fig. 3 (Fig. 2), with a suitable cartridge 2, 3 received within the device 1. Thus the figures 2 and 3 also show sectional views of a system according to the invention with the device 1 of Fig. 1 and a suitable cartridge installed therein.

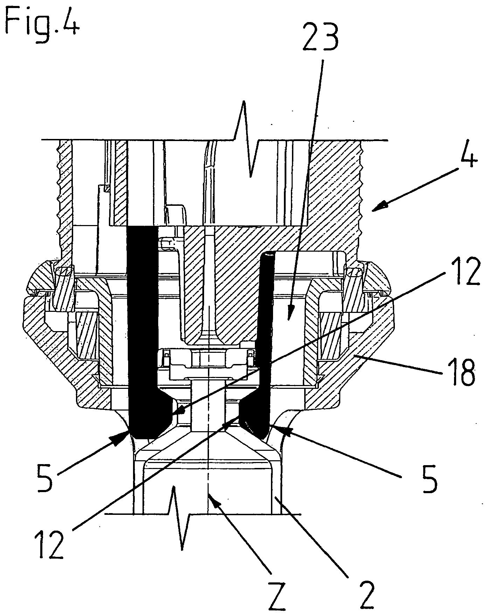

As can be seen when in addition to the Figures 1 to 3 considering Fig. 4, which shows the detail X of Fig. 3, the cartridge body 2 of the cartridge is releasably held inside the device 1 and connected to the housing 18 of the device 1 by a connector assembly 4 (first portion of the device according to the claims). The connection with the connector assembly 4 is established by arms 5 (first coupling means according to the claims) of the connector assembly 4 which engage with protrusions 12 arranged at their free ends the radial undercut formed by the neck portion of the cartridge body 2 in such a manner that, in both directions of the longitudinal axis Z of the cartridge body 2, there exists a positive connection between the cartridge body 2 and the connector assembly 4 and thus between the cartridge body 2 and the housing 18 of the device 1.

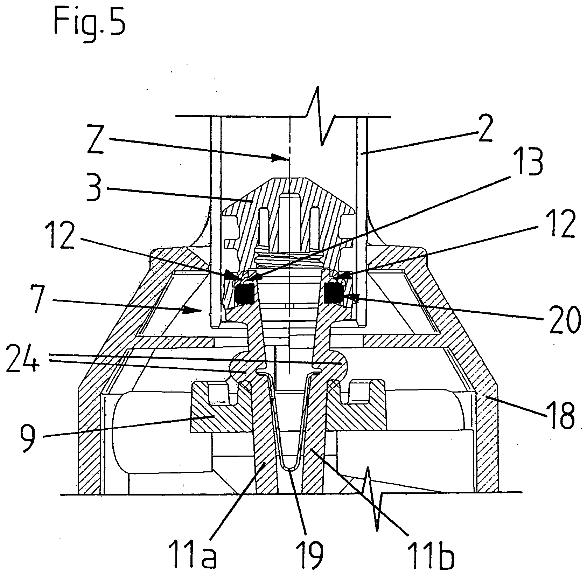

As can be seen when in addition considering Fig. 5, which shows the detail Y of Fig. 3, the piston 3 of the cartridge is releasably coupled at its backside to a pincer mechanism 7 (second coupling means according to the claims) having two resilient pincer arms 11a, 11b which are spread by a spring 19. The pincer arms 11a, 11b engage with protrusions 12 formed at their free ends a radial undercut formed by a ring 20 fixedly mounted in a central opening in the backside of the piston 3 in such a manner that in both directions of the longitudinal axis Z of the cartridge body 2 there exists a positive connection between the piston 3 and the pincer mechanism 7.

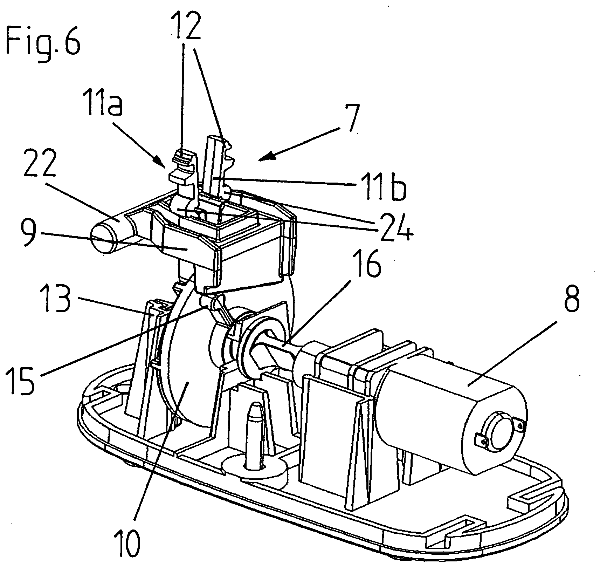

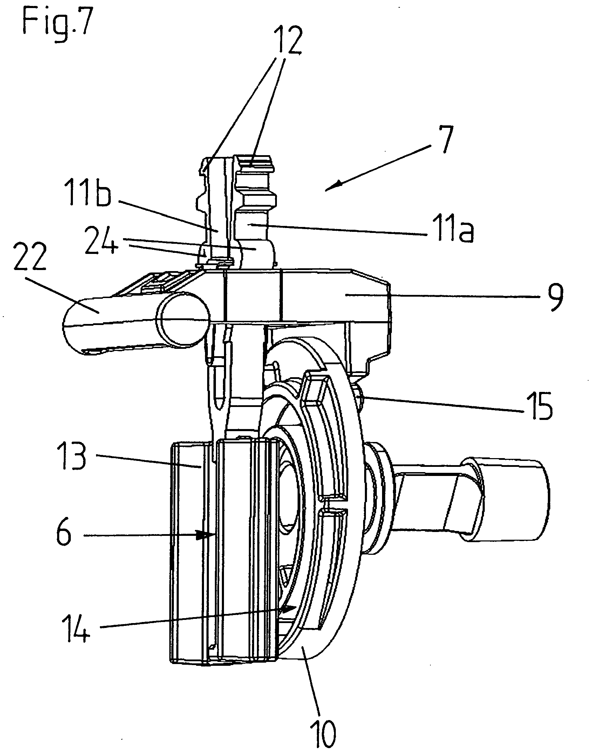

As can be seen when furthermore considering the Figures 6 and 7, which show perspective views from different directions onto the pincer mechanism 7 and the associated drive mechanism, the pincer mechanism 7 is part of a slider assembly 6 (second portion according to the claims), which is guided in guides 13 for linear movement and can be moved forwards and backwards in direction of the longitudinal axis Z of the cartridge body 2 by means of a cam wheel 10 which can be rotated by an electric motor 8 coupled to it via an rotationally flexible coupling 16.

The linkage between the slider assembly 6 and the cam wheel 10 is established by a pin 21 formed at the slider 6, which pin 21 is received in a groove 14 formed in the cam wheel 10.

When rotating the cam wheel 10 with the motor 8 clockwise and anti-clockwise, the pin 21 travels in the groove 14 between the two ends of the groove 14, thereby lifting and lowering the slider assembly 6 with its pincer mechanism 7 and, as a result, moving the piston 3 forwards and backwards in direction of the axis Z inside the cartridge body 2.

As can further be seen, the two pincer arms 11a, 11b of the pincer mechanism 7 extend through the opening of a rocker frame 9 which, by rotating the cam wheel 10 with the motor 8 clockwise and anti-clockwise within a certain angle of rotation, can at one end thereof be lifted and lowered by a notch 15 formed at the cam wheel 10, thereby tilting the rocker frame 9 around a bearing pin 22 located at its other end. Since the pincer arms 11a, 11b at there outside comprise notches 24 which under resilient pre-stress contact sliding surfaces formed at the inside of the opening of the rocker frame 9, the tilting of the rocker frame 9 around its bearing pin 22 results in an opening and closing movement of the arms 11a, 11b of pincer mechanism 7 relative to each other. Through this opening and closing of the pincer mechanism 7 the connection between the piston 3 and the slider assembly 6 can be established (opening of the pincer mechanism) and be released (closing of the pincer mechanism).

The cam wheel 10 with its groove 14 and the notch 15 is designed in such a manner that in the range of rotation in which its notch 15 is lifting and lowering the end of the rocker frame 9 and thus the pincer mechanism 7 is opened and closed, the slider assembly 6 with its pincer mechanism 7 is held by its pin 21 which is traveling in the groove 14 of the cam wheel 10 in a fully retracted position.

For loading a cartridge to be filled with insulin from an insulin reservoir into the device 1 and thus arrive at the situation shown in the Figures 2 and 3, in which the cartridge body 2 is connected via the arms 5 to the connector assembly 4 and to the housing 18 and in which the cartridge piston 3 is connected via the pincer mechanism 7 to the slider assembly 6, the connector assembly 4 is removed from the empty device 1 and is snapped with its arms 5 onto the neck portion of the cartridge body 2 of the cartridge to be filled. Subsequently, the connector assembly 4 together with the cartridge held between its arms 5 is inserted into the receiving opening 23 of the device 1, where at the end of the insertion movement it is connected with the housing 18 by hooks which can be released with a release button (not shown) after the filling operation for removal of the filled cartridge. For insertion of the cartridge into the device, the slider assembly 6 with its pincer mechanism 7 is positioned in the fully retracted position and the pincer mechanism 7 is closed, i.e. the pincer arms 11a, 11b are pushed towards each other by the rocker frame 9. While the cartridge is inserted into the receiving opening 23 of the device 1, the free ends of the pincer arms 11a, 11b are introduced into the central opening at the backside of the piston 3.

After the insertion movement has been completed, the motor 8 is activated for turning the cam wheel 10 in a first rotational direction into a rotational position in which the notch 15 is no longer supporting the end of the rocker frame 9 so that the pincer mechanism 7 opens and the pincer arms 11a, 11b engage with their protrusions 12 the radial undercut formed by the ring 20 fixed in the central opening in the backside of the piston 3.

For filling the cartridge received in the before described manner within the device 1 with insulin from an insulin reservoir (not shown), the cartridge is connected, via a cannula (not shown) extending through the connector assembly 4 and a septum arranged at the head of the cartridge, to the insulin reservoir in such a manner that the inside of the insulin reservoir and the inside of the cartridge communicate with each other while they are hermetically sealed against the outside.

After the connection with the insulin reservoir has been established, the motor 8 is activated for turning the cam wheel 10 further in the first rotational direction for moving the piston 3 forwards inside the cartridge body 2, thereby reducing the volume of the inside of the cartridge and thus displacing air from the inside of the cartridge into the insulin reservoir. When the pin 21 of the slider 6, during rotation of the cam wheel in the first rotational direction, reaches the end of the groove 14 in the cam wheel 10, the rotation of the cam wheel 10 stops and the control (not shown) of the motor 8 detects an increasing electrical current consumption of the motor 8 indicating that the fully extended position of the piston 3 has been reached.

Now the control of the motor 8 reverses the rotational direction so that the cam wheel 10 is rotated in a second rotational direction which is opposite to the first rotational direction, thereby increasing the volume of the inside of the cartridge and thus displacing insulin from the inside of the insulin reservoir into the inside of the cartridge.

Alternatively, the forward movement of the piston can be performed before connecting the cartridge to the insulin reservoir so that there is no displacement of air into the insulin reservoir.

When the pin 21 of the slider 6, during rotation of the cam wheel in the second rotational direction, reaches the opposite end of the groove 14 in the cam wheel 10, the rotation of the cam wheel 10 stops and the control of the motor 8 detects an increasing electrical current consumption of the motor 8 indicating that the fully retracted position of the piston 3 has been reached. Subsequently, the control shuts of the motor 8.

Before the pin 21 reaches, when rotating the cam wheel in the second rotational direction, the end of the groove 14, the notch 15 lifts the end of the rocker frame 9, thereby automatically releasing the connection between the piston 3 and the slider assembly 6 by closing the pincer mechanism 7. To ensure that the piston 3 is released in the fully retracted position, the cam wheel is designed in such a manner that, when the notch 15 starts lifting the end of the rocker frame 9, the piston 3 has already reached the fully retracted position.

To remove the filled cartridge from the device 1, the release button for releasing the hooks which connect the connector assembly 4 with the housing 18 is pressed and the connector assembly 4 together with the cartridge is removed from the device 1. Subsequently, the arms 5 of the connector assembly 4 are unlatched and the filled cartridge is separated from the connector assembly 4. The cartridge then is ready for its intended use.

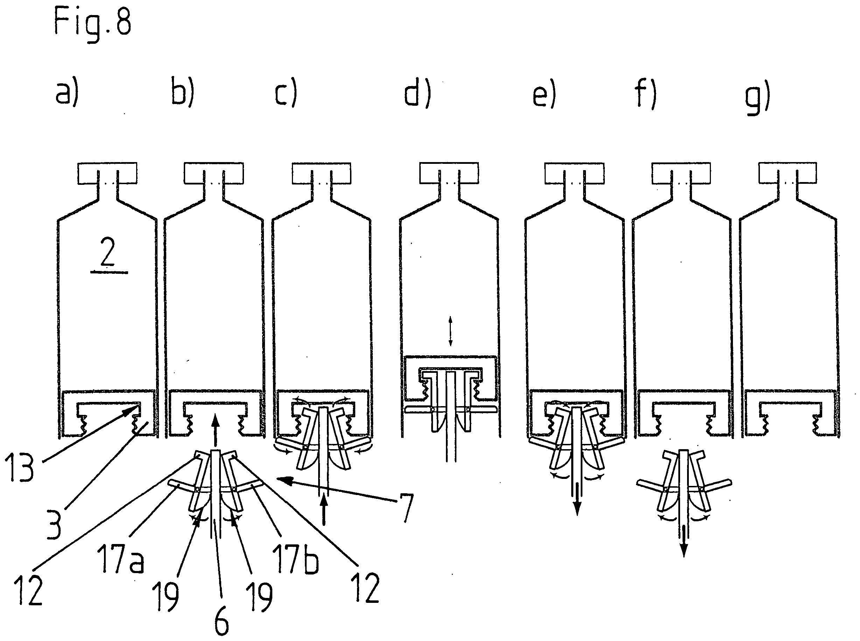

Fig. 8 a) to g) schematically illustrates the coupling and decoupling procedure of an alternative coupling concept according to the invention, which could also be used in the device described before. In this embodiment, a piston rod 6 (the second portion according to the claims) is coupled to the piston 3 of the cartridge for moving it forwards and backwards inside the cartridge body 2. The piston rod 6 at its front end comprises an lever arrangement 7 (second coupling means according to the claims) formed of two moveable levers 17a, 17b (moveable parts according to the claims) which at their free end carry protrusion 12. For the sake of good manageability, the reference signs have not been repeated in each of the situations a) to g).

As can be seen when following the different situations illustrated in Fig. 8 from situation a) to situation c), the levers 17a, 17b are held by leave springs 19 in a position that permits to introduce their free ends with the protrusions 12 into the central opening at the backside of the piston 3.

As can be seen when further considering situation d), the levers 17a, 17b are designed in such a manner that, when for coupling the piston rod 6 to the piston 3 of the cartridge the piston rod 6 is moved forward towards the piston 3 and displaces the piston 3 forwards inside the cartridge body 2, and thus the free ends of the levers 17a, 17b are introduced into the central opening at the backside of the piston 3, the levers 17a, 17b are tilted against the forces of the springs 19 through a contact with the backside of the piston 3 in such a manner that their protrusions 12 engage a radial undercut 13 in the central opening in the backside of the piston. By this, a positive connection in both directions of said longitudinal axis of the cartridge body 2 between the piston 3 and the piston rod 6 is established.

When now, as can be seen when following the different situations illustrated in Fig. 8 from situation d) to situation g), during a subsequent backward displacement of the piston 3 inside the cartridge body 2 effected by a retracting movement of the piston rod 6 the piston 3 reaches a specific retracted position within the cartridge body 2, the levers 17a, 17b are tilted, driven by the forces of the springs 19, back into their initial position, thereby removing the protrusions 12 from the radial undercut 13 in the central opening in the backside of the piston 3 so that, when the piston rod 6 is further retracted, it separates from the piston 3 without pulling the piston 3 out of the cartridge body 2.

While there are shown and described certain presently preferred embodiments of the invention relating to a device, system and method for filling a cartridge, it is to be distinctly understood that the invention is not limited thereto but may be otherwise variously embodied and practiced within the scope of the following claims. For example, in another embodiment, the device could be a liquid pump, such as an insulin pump and the first coupling means for holding the cartridge could be a cartridge compartment of the pump. In such an alternative embodiment, a plunger rod having the second coupling means as described herein can be utilized and provides the further advantage as mentioned above of preventing the plunger of the cartridge from being drawn into the cartridge by a pressure differential (such as due to a height or ambient pressure difference). In this way, a pump incorporating the disclosed second coupling means exhibits the further advantage of serving to prevent free-flow of a liquid (such as a liquid insulin solution) into a person using the pump.

## Claims

1. Device (1) for moving the piston inside a cartridge having a cartridge body (2) and a piston (3) arranged therein displaceable along a longitudinal axis (Z) of the cartridge body (2), the device (1) comprising:
- a first portion (4) having first coupling means (5) for releasably coupling the cartridge body (2) of the cartridge to the first portion (4) in such a manner that in both directions of said longitudinal axis (Z) of the cartridge body (2) there exists a positive connection and/or a frictional connection between the cartridge body (2) and the first portion (4);
- a second portion (6) having second coupling means (7, 11a, 11b, 12; 7, 12, 17a, 17b) for releasably coupling the piston (3) of the cartridge to the second portion (6) in such a manner that in both directions of said longitudinal axis (Z) of the cartridge body (2) there exists a positive connection and/or a frictional connection between the piston (3) and the second portion (6);
wherein said second portion (6) is moveable relative to the first portion (4) forward and backward between a fully retracted position and a fully extended position along a longitudinal axis in order to effect, in the intended use of the device (1), a forward and backward displacement of the piston (3) inside the cartridge body (2) coupled to the first portion (4) along said longitudinal axis (Z) of the cartridge body (2), and
wherein the second coupling means (7, 11a, 11b, 12; 7, 12, 17a, 17b) are designed in such a manner that for establishing and releasing the positive connection and/or frictional connection with the piston (3), their shape can reversibly be changed,
**characterized in that** the shape of the second coupling means (7, 11a, 11b, 12) is automatically changed when the second portion (6) is moved into the fully retracted position and/or is moved out of the fully retracted position.

2. Device (1) according to claim 1, further comprising drive means (8) for moving the first (4) and second portions (6) relative to each other.

3. Device (1) according to one of the preceding claims, wherein the second coupling means (7, 12, 17a, 17b) are designed in such a manner that their change in shape can at least partially be effected through a contact with the piston (3) and/or with the cartridge body (2).

4. Device (1) according to one of the preceding claims, wherein the second coupling means (7, 11a, 11b, 12) are designed in such a manner that their change in shape can at least partially be effected through control means (9, 15) of the device (1).

5. Device (1) according to one of the preceding claims, wherein the device (1) is designed in such a manner that the shape of the second coupling means (7, 11 a, 11b, 12; 7, 12, 17a, 17b) can or can only be changed when the first (4) and the second portions (6) assume a specific position relative to each other.

6. Device (1) according to one of the preceding claims, wherein the device (1) is designed in such a manner that the shape of the second coupling means (7, 11 a, 11b, 12) is automatically changed when the first (4) and the second portions (6) are moved into a specific position relative to each other and/or out of a specific position relative to each other.

7. Device (1) according to one of the preceding claims, wherein the device (1) is designed in such a manner that the shape of the second coupling means (7, 11 a, 11 b, 12; 7, 12, 17a, 17b) can or can only be changed into a coupled status when the cartridge body (2) of a cartridge has been coupled to the first portion (4) by means of the first coupling means (5) and/or that the shape of the second coupling means (7, 11a, 11b, 12; 7, 12, 17a, 17b) is automatically changed when, after the cartridge body (2) of a cartridge has been coupled to the first portion (4) by means of the first coupling means (5), the first coupling means (5) are released.

8. Device (1) according to one of the preceding claims, wherein the device (1) is designed in such a manner that the shape of the second coupling means (7, 11a, 11b, 12) is automatically changed when starting from the fully retracted position a forward movement of the second portion (6) is initiated.

9. Device (1) according to one of the preceding claims, wherein the second coupling means (7, 11a, 11 b, 12) comprise a pincer mechanism (11a, 11 b, 12), the shape of which can be changed through a closing and opening movement of the pincer arms (11a, 11b), and wherein the pincer mechanism (11a, 11b, 12) comprises protrusions (12) extending from the pincer arms (11a, 11 b) for engagement into radial undercuts (13) formed in a piston (3) of a cartridge to be filled.

10. Device according to claim 9, wherein the device is designed in such a manner that the pincer mechanism (11a, 11 b, 12) can be opened and closed by control means (9, 15) of the device (1) which are contacting contact surfaces (24) of the pincer arms (11a, 11 b) and are moved relative to these contact surfaces of the pincer arms (11a, 11 b) in direction of the longitudinal axis (Z) of the cartridge body (2) to open or to close the pincer mechanism (11a, 11 b, 12).

11. Device (1) according to one of the claims 9 to 10, wherein the pincer mechanism (11a, 11 b, 12) is guided in guides (13) for linear movement and can be moved forwards and backwards by means of a cam wheel (10) which can be rotated by drive means (8).

12. Device (1) according to claim 11, wherein the pincer mechanism (11a, 11 b, 12) is coupled with the cam wheel (10) via a pin (21) formed at the pincer mechanism (11a, 11 b, 12) which travels in a groove (14) formed in the cam wheel (10).

13. Device (1) according to claim 12, wherein the pincer mechanism (11a, 11 b, 12) can be opened and closed by control means (9, 15) of the device (1) which are operated by the cam wheel (10).

14. Device (1) according to claim 13, wherein the control means (9, 15) comprise a pusher (9) which can be pushed up by a notch (15) at the cam wheel (10), thereby pushing together the arms (11a, 11 b) of the pincer mechanism (11a, 11 b, 12).

15. Device (1) according to one of the claims 13 to 14, wherein the cam wheel (10) is designed in such a manner that while the pincer mechanism (11a, 11 b, 12) is opened and closed by control means (9, 15) operated by the cam wheel (10), the pincer mechanism (11a, 11b, 12) is held by the cam wheel (10) in a fully retracted position.

16. Device (1) according to one of the claims 11 to 15, wherein between the drive means (8) and the cam wheel (10) there is arranged a rotationally flexible coupling (16).

17. Device (1) according to one of the preceding claims, wherein the device is a device for filling a cartridge with a liquid from a liquid reservoir or for dispensing or dosing a liquid from a cartridge containing a liquid, in particular a liquid drug, in particular insulin.

18. A system comprising a device (1) according to one of the claims 1 to 17 and a cartridge having a cartridge body (2) and a piston (3) arranged therein displaceable along a longitudinal axis (Z) of the cartridge body (2),
wherein the cartridge body (2) is especially adapted to be releasably coupled to the first coupling means (5) of the first portion (4) of the device (1) in such a manner that in both directions of said longitudinal axis (Z) of the cartridge body (2) there exists a positive connection and/or a frictional connection between the cartridge body (2) and the first portion (4); and
wherein the piston (3) is especially adapted to be releasably coupled to the second coupling means (7, 11a, 11 b, 12; 7, 12, 17a, 17b) of the second portion (6) in such a manner that in both directions of said longitudinal axis (Z) of the cartridge body (2) there exists a positive connection and/or a frictional connection between the piston (3) and the second portion (6).

19. System according to claim 18, wherein the piston (3) of the cartridge at its back side comprises at least one radial undercut (13) and wherein the second coupling means (7, 12, 17a, 17b) of the second portion (6) of the device (1) comprise moveable parts (17a, 17b, 12) and are designed in such a manner that,
when the cartridge body (2) is coupled by the first coupling means (5) to the first portion (4) and the second portion (6) is moved towards the piston (3) and displaces the piston (3) forwards inside the cartridge body (2), the moveable parts (17a, 17b, 12) are moved, in particular against spring forces, at least partially through a contact with the piston (3) and/or with the cartridge body (2), from a position, in which there is no engagement with the at least one radial undercut (13) into a position in which they engage said at least one radial undercut (13) in such a manner that in both directions of said longitudinal axis (Z) of the cartridge body (2) there exists a positive connection between the piston (3) and the second portion (6), and
when, during a subsequent backward displacement of the piston (3) inside the cartridge body (2) effected by the second portion (6) the piston (3) reaches a specific retracted position within the cartridge body (2), the moveable parts (17a, 17b, 12) are moved, in particular by spring forces, from the position, in which they engage said at least one radial undercut (13) into the position in which there is no engagement with the at least one radial undercut (13).

20. System according to one of the claims 18 to 19, wherein the piston (3) of the cartridge at its back side comprises at least one radial undercut (13) and wherein the second coupling means (7, 11a, 11b, 12) of the second portion (6) comprise moveable parts (11a, 11 b, 12) and are designed in such a manner that,
when the cartridge body (2) is coupled by the first coupling means (5) to the first portion (4), when the second portion (6) is arranged in the fully retracted position or in a specific almost fully retracted position and when a forward movement of the second portion (6) is initiated, the moveable parts (11a, 11 b, 12), in particular by spring forces, at least partially controlled by control means (9, 15) of the device (1), are moved from a position, in which there is no engagement with the at least one radial undercut (13), into a position in which they engage said at least one radial undercut (13) in such a manner that in both directions of said longitudinal axis (Z) of the cartridge body (2) there exists a positive connection between the piston (3) and the second portion (6).

21. System according to claim 20 with a device (1) according to claim 9, wherein the second coupling means (7, 11a, 11b, 12) are designed in such a manner that, when, after the forward movement of the second portion (6) has been initiated, during a subsequent backward displacement of the piston (3) inside the cartridge body (2) effected by the second portion (6), the second portion (6) again reaches the fully retracted position or the specific almost fully retracted position, the moveable parts (11a, 11 b, 12) are moved, in particular against spring forces, at least partially controlled by control means (9, 15) of the device (1), from the position, in which they engage said at least one radial undercut (13) into the position in which there is no engagement with the at least one radial undercut (13).

22. System according to one of the claims 18 to 21 wherein the moveable parts (11a, 11 b, 12) comprise or are formed by resilient arms (11a, 11 b) of a pincer mechanism (11a, 11a, 12).

23. A method of filling a cartridge with a liquid, in particular with a liquid drug, in particular with insulin, from a liquid reservoir, comprising the steps of:
a) providing a system according to one of the claims 20 to 22 and a liquid reservoir;
b) releasably coupling the cartridge body (2) of the cartridge by means of the first coupling means (5) to the first portion (4) of the device (1) in such a manner that in both directions of said longitudinal axis (Z) of the cartridge body (2) there exists a positive connection and/or a frictional connection between the cartridge body (2) and the first portion (4);
c) releasably coupling the piston (3) of the cartridge by means of the second coupling means (7, 11a, 11b, 12; 7, 12, 17a, 17b) to the second portion (6) in such a manner that in both directions of said longitudinal axis (Z) of the cartridge body (2) there exists a positive connection and/or a frictional connection between the piston (3) and the second portion (6), wherein said connection is established through a change in shape of the second coupling means (7, 11a, 11b, 12; 7, 12, 17a, 17b);
d) connecting the cartridge and the liquid reservoir in such a manner that the inside of the liquid reservoir and the inside of the cartridge communicate with each other while they are hermetically sealed against the outside; and
e) increasing the volume of the inside of the cartridge by moving the piston (3) through a relative movement between the first (4) and second portions (6) of the device (1) backwards inside the cartridge body (2), thereby displacing liquid from the inside of the liquid reservoir into the inside of the cartridge.

24. Method according to claim 23, wherein, before the volume of the inside of the cartridge is increased by moving the piston (3) through a relative movement between the first (4) and second portions (6) of the device (1) backwards inside the cartridge body (2) for displacing liquid from the inside of the liquid reservoir into the inside of the cartridge, the volume of the inside of the cartridge is reduced by moving the piston (3) through a relative movement between the first (4) and second portions (6) of the device (1) forwards inside the cartridge body (2).

## Patentansprüche

1. Vorrichtung (1) zum Bewegen eines Kolbens im Inneren einer Kartusche, die einen Kartuschenkörper (2) und einen darin angeordneten Kolben (3) aufweist, der entlang einer Längsachse (Z) des Kartuschenkörpers (2) verschiebbar ist, wobei die Vorrichtung (1) umfasst:
- einen ersten Teil (4) mit ersten Kopplungsmitteln (5) zum lösbaren Koppeln des Kartuschenkörpers (2) der Kartusche an den ersten Teil (4) auf eine solche Weise, dass in beiden Richtungen der Längsachse (Z) des Kartuschenkörpers (2) eine formschlüssige Verbindung und/oder eine Reibungsverbindung zwischen dem Kartuschenkörper (2) und dem ersten Teil (4) besteht;
- einen zweiten Teil (6) mit zweiten Kopplungsmitteln (7, 11a, 11b, 12; 7, 12, 17a, 17b) zum lösbaren Koppeln des Kolbens (3) der Kartusche an den zweiten Teil (6) auf eine solche Weise, dass in beiden Richtungen der Längsachse (Z) des Kartuschenkörpers (2) eine formschlüssige Verbindung und/oder eine Reibungsverbindung zwischen dem Kolben (3) und dem zweiten Teil (6) besteht;
wobei der zweite Teil (6) relativ zu dem ersten Teil (4) vorwärts und rückwärts zwischen einer vollständig zurückgezogenen Stellung und einer vollständig vorgeschobenen Stellung entlang einer Längsachse beweglich ist, um bei der vorgesehenen Verwendung der Vorrichtung (1) eine Vorwärts- und Rückwärtsverschiebung des Kolbens (3) im Inneren des an den ersten Teil (4) gekoppelten Kartuschenkörpers (2) entlang der Längsachse (Z) des Kartuschenkörpers (2) zu bewirken, und wobei die zweiten Kopplungsmittel (7, 11a, 11b, 12; 7, 12, 17a, 17b) so gestaltet sind, dass zum Bilden und Lösen der formschlüssigen Verbindung und/oder Reibungsverbindung mit dem Kolben (3) ihre Form reversibel verändert werden kann,
**dadurch gekennzeichnet, dass** die Form der zweiten Kopplungsmittel (7, 11a, 11b, 12) automatisch verändert wird, wenn der zweite Teil (6) in die vollständig zurückgezogene Stellung bewegt wird und/oder aus der vollständig zurückgezogenen Stellung herausbewegt wird.

2. Vorrichtung (1) gemäß Anspruch 1, ferner umfassend Antriebsmittel (8) zum Bewegen des ersten (4) und des zweiten Teils (6) relativ zueinander.

3. Vorrichtung (1) gemäß einem der vorstehenden Ansprüche, wobei die zweiten Kopplungsmittel (7, 12, 17a, 17b) so gestaltet sind, dass ihre Formveränderung wenigstens teilweise durch einen Kontakt mit dem Kolben (3) und/oder mit dem Kartuschenkörper (2) bewirkt werden kann.

4. Vorrichtung (1) gemäß einem der vorstehenden Ansprüche, wobei die zweiten Kopplungsmittel (7, 11a, 11b, 12) so gestaltet sind, dass ihre Formveränderung wenigstens teilweise durch Steuermittel (9, 15) der Vorrichtung (1) bewirkt werden kann.

5. Vorrichtung (1) gemäß einem der vorstehenden Ansprüche, wobei die Vorrichtung (1) so gestaltet ist, dass die Form der zweiten Kopplungsmittel (7, 11a, 11b, 12; 7, 12, 17a, 17b) verändert werden kann oder nur verändert werden kann, wenn der erste (4) und der zweite Teil (6) eine spezifische Stellung zueinander einnehmen.

6. Vorrichtung (1) gemäß einem der vorstehenden Ansprüche, wobei die Vorrichtung (1) so gestaltet ist, dass die Form der zweiten Kopplungsmittel (7, 11a, 11b, 12) automatisch verändert wird, wenn der erste (4) und der zweite Teil (6) in eine spezifische Stellung relativ zueinander bewegt werden und/oder aus einer spezifischen Stellung relativ zueinander herausbewegt werden.

7. Vorrichtung (1) gemäß einem der vorstehenden Ansprüche, wobei die Vorrichtung (1) so gestaltet ist, dass die Form der zweiten Kopplungsmittel (7, 11a 11b, 12; 7, 12, 17a, 17b) in einen gekoppelten Zustand verändert werden kann oder nur verändert werden kann, wenn der Kartuschenkörper (2) einer Kartusche mithilfe der ersten Kopplungsmittel (5) an den ersten Teil gekoppelt worden ist und/oder dass die Form der zweiten Kopplungsmittel (7, 11a, 11b, 12; 7, 12, 17a, 17b) automatisch verändert wird, wenn, nachdem der Kartuschenkörper (2) einer Kartusche mithilfe der ersten Kopplungsmittel (5) an den ersten Teil gekoppelt worden ist, die ersten Kopplungsmittel (5) gelöst werden.

8. Vorrichtung (1) gemäß einem der vorstehenden Ansprüche, wobei die Vorrichtung (1) so gestaltet ist, dass die Form der zweiten Kopplungsmittel (7, 11a, 11b, 12) automatisch verändert wird, wenn ausgehend von der vollständig zurückgezogenen Stellung eine Vorwärtsbewegung des zweiten Teils (6) begonnen wird.

9. Vorrichtung (1) gemäß einem der vorstehenden Ansprüche, wobei die zweiten Kopplungsmittel (7, 11a, 11b, 12) einen Zangenmechanismus (11a 11b, 12) umfassen, dessen Form durch eine Schließ- und Öffnungsbewegung der Zangenarme (11a 11b) verändert werden kann, und wobei der Zangenmechanismus (11a 11b, 12) Vorsprünge, die von den Zangenarmen (11a 11b) vorragen, für den Eingriff in radiale Hohlkehlen (13) umfasst, die in einem Kolben (3) einer zu füllenden Kartusche gebildet sind.

10. Vorrichtung (1) gemäß Anspruch 9, wobei die Vorrichtung so gestaltet ist, dass der Zangenmechanismus (11a 11b, 12) durch Steuermittel (9, 15) der Vorrichtung (1) geöffnet und geschlossen werden können, die in Kontakt mit Kontaktoberflächen (24) der Zangenarme (11a, 11b) stehen und relativ zu diesen Kontaktoberflächen der Zangenarme (11a 11b) in der Richtung der Längsachse (Z) des Kartuschenkörpers (2) bewegt werden, um den Zangenmechanismus (11a, 11b, 12) zu öffnen oder zu schließen.

11. Vorrichtung (1) gemäß einem der Ansprüche 9 bis 10, wobei der Zangenmechanismus (11a, 11b, 12) in Führungen (13) für lineare Bewegung geführt wird und mithilfe eines Nockenrads (10), das durch Antriebsmittel (8) gedreht werden kann, vorwärts und rückwärts bewegt werden kann.

12. Vorrichtung (1) gemäß Anspruch 11, wobei der Zangenmechanismus (11a 11b, 12) über einen Stift (21), der an dem Zangenmechanismus (11a, 11b, 12) gebildet ist und sich in einer in dem Nockenrad (10) gebildeten Rille (14) bewegt, an das Nockenrad (10) gekoppelt ist.

13. Vorrichtung (1) gemäß Anspruch 12, wobei der Zangenmechanismus (11a 11b, 12) durch Steuermittel (9, 15) der Vorrichtung (1), die von dem Nockenrad (10) betrieben werden, geöffnet und geschlossen werden kann.

14. Vorrichtung (1) gemäß Anspruch 13, wobei das Steuermittel (9, 15) einen Drücker (9) umfasst, der von einer Nut (15) an dem Nockenrad (10) nach oben gedrückt werden kann und dadurch die Arme (11a, 11b) des Zangenmechanismus (11a 11b, 12) zusammendrückt .

15. Vorrichtung (1) gemäß einem der Ansprüche 13 bis 14, wobei das Nockenrad (10) so gestaltet ist, dass während der Zangenmechanismus (11a, 11b, 12) durch von dem Nockenrad (10) betriebene Steuermittel (9, 15) geöffnet und geschlossen wird, der Zangenmechanismus (11a, 11b, 12) von dem Nockenrad (10) in einer vollständig zurückgezogenen Stellung gehalten wird.

16. Vorrichtung (1) gemäß einem der Ansprüche 11 bis 15, wobei zwischen dem Antriebsmittel (8) und dem Nockenrad (10) eine drehelastische Kupplung (16) angeordnet ist.

17. Vorrichtung (1) gemäß einem der vorstehenden Ansprüche, wobei die Vorrichtung eine Vorrichtung zum Füllen einer Kartusche mit einer Flüssigkeit aus einem Flüssigkeitsreservoir oder zum Ausgeben oder Dosieren einer Flüssigkeit aus einer Kartusche ist, die eine Flüssigkeit enthält, insbesondere ein flüssiges Arzneimittel, insbesondere Insulin.

18. System umfassend eine Vorrichtung (1) gemäß einem der Ansprüche 1 bis 17 und eine Kartusche mit einem Kartuschenkörper (2) und einem Kolben (3), der darin entlang einer Längsachse (Z) des Kartuschenkörpers (2) verschiebbar angeordnet ist,
wobei der Kartuschenkörper (2) insbesondere dafür ausgelegt ist, auf eine solche Weise lösbar an die ersten Kopplungsmittel (5) des ersten Teils (4) der Vorrichtung (1) gekoppelt zu werden, dass in beiden Richtungen der Längsachse (Z) des Kartuschenkörpers (2) eine formschlüssige Verbindung und/oder eine Reibungsverbindung zwischen dem Kartuschenkörper (2) und dem ersten Teil (4) besteht; und
wobei der Kolben (3) insbesondere dafür ausgelegt ist, auf eine solche Weise lösbar an die zweiten Kopplungsmittel (7, 11a, 11b, 12; 7, 12, 17a, 17b) des zweiten Teils (6) gekoppelt zu werden, dass in beiden Richtungen der Längsachse (Z) des Kartuschenkörpers (2) eine formschlüssige Verbindung und/oder eine Reibungsverbindung zwischen dem Kolben (3) und dem zweiten Teil (6) besteht.

19. System gemäß Anspruch 18, wobei der Kolben (3) der Kartusche an seiner Rückseite wenigstens eine radiale Hohlkehle (13) umfasst und wobei die zweiten Kopplungsmittel (7, 12, 17a, 17b) des zweiten Teils (6) der Vorrichtung (1) bewegliche Teile (17a, 17b, 12) umfassen und so gestaltet sind, dass
wenn der Kartuschenkörper (2) durch die ersten Kopplungsmittel (5) an den ersten Teil (4) gekoppelt ist und der zweite Teil (6) zu dem Kolben (3) bewegt wird und den Kolben (3) in dem Kartuschenkörper (2) vorwärts verschiebt, die beweglichen Teile (17a, 17b, 12) insbesondere gegen Federkräfte wenigstens teilweise durch einen Kontakt mit dem Kolben (3) und/oder mit dem Kartuschenkörper von einer Stellung, in der kein Eingriff mit der wenigstens einen radialen Hohlkehle (13) besteht, in eine Stellung bewegt werden, in der sie mit der wenigstens einen radialen Hohlkehle (13) auf solche Weise in Eingriff stehen, dass in beiden Richtungen der Längsachse (Z) des Kartuschenkörpers (2) eine formschlüssige Verbindung zwischen dem Kolben (3) und dem zweiten Teil (6) besteht, und
wenn während einer nachfolgenden Rückwärtsverschiebung des Kolbens (3) in dem Kartuschenkörper (2), die von dem zweiten Teil (6) bewirkt wird, der Kolben (3) eine spezifische zurückgezogene Stellung in dem Kartuschenkörper (2) erreicht, die beweglichen Teile (17a, 17b, 12) insbesondere durch Federkräfte von der Stellung, in der sie mit der wenigstens einen radialen Hohlkehle (13) in Eingriff stehen, in die Stellung bewegt werden, in der kein Eingriff mit der wenigstens einen radialen Hohlkehle (13) besteht.

20. System gemäß einem der Ansprüche 18 bis 19, wobei der Kolben (3) der Kartusche an seiner Rückseite wenigstens eine radiale Hohlkehle (13) umfasst und wobei die zweiten Kopplungsmittel (7, 11a, 11b, 12) des zweiten Teils (6) bewegliche Teile (11a, 11b, 12) umfassen und so gestaltet sind, dass
wenn der Kartuschenkörper (2) durch die ersten Kopplungsmittel (5) an den ersten Teil (4) gekoppelt ist, wenn der zweite Teil (6) in der vollständig zurückgezogenen Stellung oder in einer spezifischen, beinahe vollständig zurückgezogenen Stellung angeordnet ist, und wenn eine Vorwärtsbewegung des zweiten Teils (6) begonnen wird, die beweglichen Teile (11a 11b, 12) insbesondere durch Federkräfte, wenigstens teilweise durch Steuermittel (9, 15) der Vorrichtung (1) gesteuert, von einer Stellung, in der kein Eingriff mit der wenigstens einen radialen Hohlkehle (13) besteht, in eine Stellung bewegt werden, in der sie mit der wenigstens einen radialen Hohlkehle (13) auf solche Weise in Eingriff stehen, dass in beiden Richtungen der Längsachse (Z) des Kartuschenkörpers (2) eine formschlüssige Verbindung zwischen dem Kolben (3) und dem zweiten Teil (6) besteht.

21. System gemäß Anspruch 20 mit einer Vorrichtung (1) gemäß Anspruch 9, wobei die zweiten Kopplungsmittel (7, 11a, 11b, 12) so gestaltet sind, dass, wenn nach dem Beginnen der Vorwärtsbewegung des zweiten Teils (6) während einer nachfolgenden Rückwärtsverschiebung des Kolbens (3) in dem Kartuschenkörper (2), die von dem zweiten Teil (6) bewirkt wird, der zweite Teil (6) wieder die vollständig zurückgezogene Stellung oder die spezifische, beinahe vollständig zurückgezogene Stellung erreicht, die beweglichen Teile (11a 11b, 12) insbesondere gegen Federkräfte, wenigstens teilweise durch Steuermittel (9, 15) der Vorrichtung (1) gesteuert, von der Stellung, in der sie in Eingriff mit der wenigstens einen radialen Hohlkehle (13) stehen, in die Stellung bewegt werden, in der kein Eingriff mit der wenigstens einen radialen Hohlkehle (13) besteht.

22. System gemäß einem der Ansprüche 18 bis 21, wobei die beweglichen Teile (11a, 11b, 12) biegsame Arme (11a 11b) eines Zangenmechanismus (11a, 11b, 12) umfassen oder daraus bestehen.

23. Verfahren zum Füllen einer Kartusche mit einer Flüssigkeit, insbesondere mit einem flüssigen Arzneimittel, insbesondere mit Insulin, aus einem Flüssigkeitsreservoir, umfassend die Schritte:
a) Bereitstellen eines Systems gemäß einem der Ansprüche 20 bis 22 und eines Flüssigkeitsreservoirs;
b) lösbares Koppeln des Kartuschenkörpers (2) der Kartusche an den ersten Teil (4) der Vorrichtung (1) mithilfe der ersten Kopplungsmittel (5) auf eine solche Weise, dass in beiden Richtungen der Längsachse (Z) des Kartuschenkörpers (2) eine formschlüssige Verbindung und/oder eine Reibungsverbindung zwischen dem Kartuschenkörper (2) und dem ersten Teil (4) besteht;
c) lösbares Koppeln des Kolbens (3) der Kartusche an den zweiten Teil (6) mithilfe der zweiten Kopplungsmittel (7, 11a, 11b, 12; 7, 12, 17a, 17b) auf eine solche Weise, dass in beiden Richtungen der Längsachse (Z) des Kartuschenkörpers (2) eine formschlüssige Verbindung und/oder eine Reibungsverbindung zwischen dem Kolben (3) und dem zweiten Teil (6) besteht, wobei die Verbindung durch eine Veränderung der Form der zweiten Kopplungsmittel (7, 11a, 11b, 12; 7, 12, 17a, 17b) gebildet wird;
d) Verbinden der Kartusche und des Flüssigkeitsreservoirs auf eine solche Weise, dass der Innenraum des Flüssigkeitsreservoirs und der Innenraum der Kartusche miteinander kommunizieren, während sie hermetisch gegen den Außenraum abgedichtet sind; und
e) Erhöhen des Volumens des Innenraums der Kartusche durch Bewegen des Kolbens (3) durch eine relative Bewegung zwischen dem ersten (4) und dem zweiten Teil (6) der Vorrichtung (1) rückwärts in dem Kartuschenkörper (2), wodurch Flüssigkeit von dem Innenraum des Flüssigkeitsreservoirs in den Innenraum der Kartusche bewegt wird.

24. Verfahren gemäß Anspruch 23, wobei, bevor das Volumen des Innenraums der Kartusche durch Bewegen des Kolbens (3) durch eine relative Bewegung zwischen dem ersten (4) und dem zweiten Teil (6) der Vorrichtung (1) rückwärts in dem Kartuschenkörper (2) erhöht wird, um Flüssigkeit von dem Innenraum des Flüssigkeitsreservoirs in den Innenraum der Kartusche zu bewegen, das Volumen des Innenraums der Kartusche durch Bewegen des Kolbens (3) durch eine relative Bewegung zwischen dem ersten (4) und dem zweiten Teil (6) der Vorrichtung (1) vorwärts in dem Kartuschenkörper (2) verringert wird.

## Revendications

1. Dispositif (1) pour déplacer le piston à l'intérieur d'une cartouche ayant un corps de cartouche (2) et dans lequel est disposé un piston (3) qui peut être déplacé le long d'un axe longitudinal (Z) du corps de cartouche (2), le dispositif (1) comprenant :
- une première partie (4) ayant des premiers moyens de couplage (5) pour coupler de manière amovible le corps de cartouche (2) de la cartouche à la première partie (4) de telle manière que, dans les deux directions dudit axe longitudinal (Z) du corps de cartouche (2), il existe une liaison positive et/ou une liaison par friction entre le corps de cartouche (2) et la première partie (4) ;
- une seconde partie (6) ayant des seconds moyens de couplage (7, 11a, 11b, 12 ; 7, 12, 17a, 17b) pour coupler de manière amovible le piston (3) de la cartouche à la seconde partie (6) de telle manière que, dans les deux directions dudit axe longitudinal (Z) du corps de cartouche (2), il existe une liaison positive et/ou une liaison par friction entre le piston (3) et la seconde partie (6) ;
dans lequel ladite seconde partie (6) peut être déplacée par rapport à la première partie (4) vers l'avant et vers l'arrière entre une position complètement rentrée et une position complètement déployée le long d'un axe longitudinal afin d'effectuer, lors de l'utilisation prévue du dispositif (1), un déplacement vers l'avant et vers l'arrière du piston (3) à l'intérieur du corps de cartouche (2) couplé à la première partie (4) le long dudit axe longitudinal (Z) du corps de cartouche (2), et
dans lequel les seconds moyens de couplage (7, 11a 11b, 12 ; 7, 12, 17a, 17b) sont conçus de telle manière que, pour établir et libérer la liaison positive et/ou la liaison par friction avec le piston (3), leur forme puisse être changée de manière réversible,
**caractérisé en ce que** la forme des seconds moyens de couplage (7, 11a 11b, 12) est changée automatiquement lorsque la seconde partie (6) est déplacée jusqu'à la position complètement rentrée et/ou quitte la position complètement rentrée.

2. Dispositif (1) selon la revendication 1, comprenant en outre un moyen d'entraînement (8) pour déplacer la première (4) et la seconde (6) partie l'une par rapport à l'autre.

3. Dispositif (1) selon l'une quelconque des revendications précédentes, dans lequel les seconds moyens de couplage (7, 12, 17a, 17b) sont conçus de telle manière que le changement de leur forme puisse au moins partiellement être effectué au moyen d'un contact avec le piston (3) et/ou avec le corps de cartouche (2).

4. Dispositif (1) selon l'une quelconque des revendications précédentes, dans lequel les seconds moyens de couplage (7, 11a 11b, 12) sont conçus de telle manière que le changement de leur forme puisse au moins partiellement être effectué par des moyens de commande (9, 15) du dispositif (1).

5. Dispositif (1) selon l'une quelconque des revendications précédentes, dans lequel le dispositif (1) est conçu de telle manière que la forme des seconds moyens de couplage (7, 11a, 11b, 12 ; 7, 12, 17a, 17b) puisse, ou puisse seulement, être changée lorsque la première (4) et la seconde (6) partie prennent une position spécifique l'une par rapport à l'autre.

6. Dispositif (1) selon l'une quelconque des revendications précédentes, dans lequel le dispositif (1) est conçu de telle manière que la forme des seconds moyens de couplage (7, 11a, 11b, 12) soit changée automatiquement lorsque la première (4) et la seconde (6) partie sont déplacées jusqu'à une position spécifique l'une par rapport à l'autre et/ou quittent une position spécifique l'une par rapport à l'autre.

7. Dispositif (1) selon l'une quelconque des revendications précédentes, dans lequel le dispositif (1) est conçu de telle manière que la forme des seconds moyens de couplage (7, 11a, 11b, 12 ; 7, 12, 17a, 17b) puisse, ou puisse seulement, être changée dans un état couplé lorsque le corps de cartouche (2) d'une cartouche a été couplé à la première partie (4) par les premiers moyens de couplage (5) et/ou que la forme des seconds moyens de couplage (7, 11a, 11b, 12 ; 7, 12, 17a, 17b) soit changée automatiquement lorsque, après que le corps de cartouche (2) d'une cartouche a été couplé à la première partie (4) par les premiers moyens de couplage (5), les premiers moyens de couplage (5) sont libérés.

8. Dispositif (1) selon l'une quelconque des revendications précédentes, dans lequel le dispositif (1) est conçu de telle manière que la forme des seconds moyens de couplage (7, 11a, 11b, 12) soit changée automatiquement lorsque, lors du départ de la position complètement rentrée, un mouvement vers l'avant de la seconde partie (6) est amorcé.

9. Dispositif (1) selon l'une quelconque des revendications précédentes, dans lequel les seconds moyens de couplage (7, 11a, 11b, 12) comprennent un mécanisme à pinces (11a, 11b, 12) dont la forme peut être changée au moyen d'un mouvement de fermeture et d'ouverture des bras de pince (11a 11b) et dans lequel le mécanisme à pinces (11a, 11b, 12) comprend des saillies (12) s'étendant depuis les bras de pince (11a, 11b) pour venir en contact dans des gorges radiales (13) formées dans un piston (3) d'une cartouche qui doit être remplie.

10. Dispositif selon la revendication 9, dans lequel le dispositif est conçu de telle manière que le mécanisme à pinces (11a, 11b, 12) puisse être ouvert et fermé par des moyens de commande (9, 15) du dispositif (1) qui viennent en contact avec des surfaces de contact (24) des bras de pince (11a 11b) et sont déplacés par rapport à ces surfaces de contact des bras de pince (11a 11b) dans une direction de l'axe longitudinal (Z) du corps de cartouche (2) pour ouvrir ou pour fermer le mécanisme à pinces (11a 11b, 12).

11. Dispositif (1) selon l'une quelconque des revendications 9 à 10, dans lequel le mécanisme à pinces (11a, 11b, 12) est guidé dans des guides (13) pour un mouvement linéaire et peut être déplacé vers l'avant et vers l'arrière au moyen d'une roue à came (10) qui peut être tournée par un moyen d'entraînement (8).

12. Dispositif (1) selon la revendication 11, dans lequel le mécanisme à pinces (11a, 11b, 12) est couplé à la roue à came (10) par le biais d'une broche (21) formée au niveau du mécanisme à pinces (11a 11b, 12) qui se déplace dans une rainure (14) formée dans la roue à came (10).

13. Dispositif (1) selon la revendication 12, dans lequel le mécanisme à pinces (11a, 11b, 12) peut être ouvert ou fermé par les moyens de commande (9, 15) du dispositif (1) qui sont actionnés par la roue à came (10).

14. Dispositif (1) selon la revendication 13, dans lequel les moyens de commande (9, 15) comprennent un poussoir (9) qui peut être poussé vers le haut par une encoche (15) au niveau de la roue à came (10), ce qui permet de pousser conjointement les bras (11a, 11b) du mécanisme à pinces (11a, 11b, 12).

15. Dispositif (1) selon l'une quelconque des revendications 13 à 14, dans lequel la roue à came (10) est conçue de telle manière que, tandis que le mécanisme à pinces (11a, 11b, 12) est ouvert et fermé par des moyens de commande (9, 15) actionnés par la roue à came (10), le mécanisme à pinces (11a, 11b, 12) est retenu par la roue à came (10) dans une position complètement rentrée.

16. Dispositif (1) selon l'une quelconque des revendications 11 à 15, dans lequel, entre les moyens d'entraînement (8) et la roue à came (10), est disposé un couplage flexible en rotation (16).

17. Dispositif (1) selon l'une quelconque des revendications précédentes, dans lequel le dispositif est un dispositif pour remplir une cartouche avec un liquide provenant d'un réservoir de liquide ou pour distribuer ou doser un liquide provenant d'une cartouche contenant un liquide, en particulier un médicament liquide, en particulier de l'insuline.

18. Système comprenant un dispositif (1) selon l'une quelconque des revendications 1 à 17 et une cartouche ayant un corps de cartouche (2) et dans lequel est disposé un piston (3) qui peut être déplacé le long d'un axe longitudinal (Z) du corps de cartouche (2),
dans lequel le corps de cartouche (2) est, en particulier, conçu pour être couplé de manière amovible à des premiers moyens de couplage (5) de la première partie (4) du dispositif (1) de telle manière que, dans les deux directions dudit axe longitudinal (Z) du corps de cartouche (2), il existe une liaison positive et/ou une liaison par friction entre le corps de cartouche (2) et la première partie (4) ; et
dans lequel le piston (3) est, en particulier, conçu pour être couplé de manière amovible à des seconds moyens de couplage (7, 11a, 11b, 12 ; 7, 12, 17a, 17b) de la seconde partie (6) de telle manière que, dans les deux directions dudit axe longitudinal (Z) du corps de cartouche (2), il existe une liaison positive et/ou une liaison par friction entre le piston (3) et la seconde partie (6).

19. Système selon la revendication 18, dans lequel le piston (3) de la cartouche au niveau de son côté arrière comprend au moins une gorge radiale (13) et dans lequel les second moyens de couplage (7, 12, 17a, 17b) de la seconde partie (6) du dispositif (1) comprennent des parties mobiles (17a, 17b, 12) et sont conçus de telle manière que,
lorsque le corps de cartouche (2) est couplé par les premiers moyens de couplage (5) à la première partie (4) et que la seconde partie (6) est déplacée vers le piston (3) et déplace le piston (3) vers l'avant à l'intérieur du corps de cartouche (2), les parties mobiles (17a, 17b, 12) soient déplacées, en particulier à l'encontre de forces de ressort, au moins partiellement au moyen d'un contact avec le piston (3) et/ou avec le corps de cartouche (2), depuis une position dans laquelle il n'y a pas de contact avec la ou les gorges radiales (13), jusqu'à une position dans laquelle elles viennent en contact avec ladite ou lesdites gorges radiales (13) de telle manière que, dans les deux directions dudit axe longitudinal (Z) du corps de cartouche (2), il existe une liaison positive entre le piston (3) et la seconde partie (6), et lorsque, pendant un déplacement ultérieur vers l'arrière du piston (3) à l'intérieur du corps de cartouche (2) effectué par la seconde partie (6), le piston (3) atteint une position rentrée spécifique à l'intérieur du corps de cartouche (2), les parties mobiles (17a, 17b, 12) soient déplacées, en particulier par des forces de ressort, depuis la position, dans laquelle elles viennent en contact avec ladite ou lesdites gorges radiales (13), jusqu'à la position dans laquelle il n'y a pas de contact avec la ou les gorges radiales (13).

20. Système selon l'une quelconque des revendications 18 à 19, dans lequel le piston (3) de la cartouche au niveau de son côté arrière comprend au moins une gorge radiale (13) et dans lequel les second moyens de couplage (7, 11a, 11b, 12) de la seconde partie (6) comprennent des parties mobiles (11a, 11b, 12) et sont conçus de telle manière que,
lorsque le corps de cartouche (2) est couplé par les premiers moyens de couplage (5) à la première partie (4), lorsque la seconde partie (6) est mise dans la position complètement rentrée ou dans une position presque complètement rentrée spécifique et lorsqu'un mouvement vers l'avant de la seconde partie (6) est amorcé, les parties mobiles (11a, 11b, 12), en particulier sous l'effet de forces de ressort, au moins partiellement actionnées par des moyens de commande (9, 15) du dispositif (1) soient déplacées depuis une position dans laquelle il n'y a pas de contact avec la ou les gorges radiales (13), jusqu'à une position dans laquelle elles viennent en contact avec ladite ou lesdites gorges radiales (13) de telle manière que, dans les deux directions dudit axe longitudinal (Z) du corps de cartouche (2), il existe une liaison positive entre le piston (3) et la seconde partie (6).

21. Système selon la revendication 20 avec un dispositif (1) selon la revendication 9, dans lequel les seconds moyens de couplage (7, 11a, 11b, 12) sont conçus de telle manière que, lorsque, après que le mouvement vers l'avant de la seconde partie (6) a été amorcé, pendant un déplacement ultérieur vers l'arrière du piston (3) à l'intérieur du corps de cartouche (2) effectué par la seconde partie (6), la seconde partie (6) atteint à nouveau la position complètement rentrée ou la position presque complètement rentrée spécifique, les parties mobiles (11a 11b, 12) soient déplacées, en particulier à l'encontre de forces de ressort, au moins partiellement actionnées par des moyens de commande (9, 15) du dispositif (1), depuis la position dans laquelle elles viennent en contact avec ladite ou lesdites gorges radiales (13), jusqu'à la position dans laquelle il n'y a pas de contact avec la ou les gorges radiales (13).

22. Système selon l'une quelconque des revendication 18 à 21, dans lequel les parties mobiles (11a 11b, 12) comprennent, ou sont formées par, des bras élastiques (11a, 11b) d'un mécanisme à pinces (11a, 11b, 12).

23. Procédé de remplissage d'une cartouche avec un liquide, en particulier avec un médicament liquide, en particulier avec de l'insuline, provenant d'un réservoir de liquide, comprenant les étapes consistant à :
a) fournir un système selon l'une quelconque des revendications 20 à 22 et un réservoir de liquide ;
b) coupler de manière amovible le corps de cartouche (2) de la cartouche par les premiers moyens de couplage (5) à la première partie (4) du dispositif (1) de telle manière que, dans les deux directions dudit axe longitudinal (Z) du corps de cartouche (2), il existe une liaison positive et/ou une liaison par friction entre le corps de cartouche (2) et la première partie (4) ;
c) coupler de manière amovible le piston (3) de la cartouche par les seconds moyens de couplage (7, 11a, 11b, 12 ; 7, 12, 17a, 17b) à la seconde partie (6) de telle manière que, dans les deux directions dudit axe longitudinal (Z) du corps de cartouche (2), il existe une liaison positive et/ou une liaison par friction entre le piston (3) et la seconde partie (6), dans lequel ladite liaison est établie au moyen d'un changement de la forme des seconds moyens de couplage (7, 11a, 11b, 12 ; 7, 12, 17a, 17b) ;
d) raccorder la cartouche et le réservoir de liquide de telle manière que l'intérieur du réservoir de liquide et l'intérieur de la cartouche communiquent l'un avec l'autre pendant qu'ils sont scellés hermétiquement par rapport à l'extérieur ; et
e) augmenter le volume de l'intérieur de la cartouche par déplacement du piston (3) au moyen d'un mouvement relatif entre la première (4) et la seconde (6) partie du dispositif (1) vers l'arrière à l'intérieur du corps de cartouche (2), ce qui permet de déplacer un liquide depuis l'intérieur du réservoir de liquide jusqu'à l'intérieur de la cartouche.

24. Procédé selon la revendication 23, dans lequel, avant que le volume de l'intérieur de la cartouche ne soit augmenté par déplacement du piston (3) au moyen d'un mouvement relatif entre la première (4) et la seconde (6) partie du dispositif (1) vers l'arrière à l'intérieur du corps de cartouche (2) pour déplacer un liquide depuis l'intérieur du réservoir de liquide jusqu'à l'intérieur de la cartouche, le volume de l'intérieur de la cartouche est réduit par déplacement du piston (3) au moyen d'un mouvement relatif entre la première (4) et la seconde (6) partie du dispositif (1) vers l'avant à l'intérieur du corps de cartouche (2).
